# EUROPEAN PATENT APPLICATION

(11) **EP 3 981 252 A1**
(43) Date of publication of application: **13.04.2022**
(21) Application number: 20200498.2
(22) Date of filing: 07.10.2020
(51) Int. Cl.: A01N 59/20, A01N 25/30, A01N 25/34, A01P 1/00, A41D 13/11, A41D 31/30, A62B 18/02

(54) **ANTIMICROBIAL FACEMASK**

(71) Applicant: Chemical Intelligence Limited, Harston Cambridge CB22 7QJ (GB)
(72) Inventor: GROS, Robert, Harston, Cambridge CB22 7QJ (GB); WILKINSON, Mark, Harston, Cambridge CB22 7QJ (GB); WIGHT, Paul, Harston, Cambridge CB22 7QJ (GB)
(74) Representative: HGF

(57) **Abstract**

An antimicrobial facemask comprising certain biocides in conjunction with certain surfactants. In particular the facemask comprises a non-woven fabric or material incorporating a water soluble copper salt and a zwitterionic surfactant, or alkylsulfate. There is also provided a process for treating a fabric suitable for a facemask construction with an aqueous solution of the desired formulation.

## Description

### FIELD OF INVENTION

The present invention relates to an antimicrobial facemask, use of the antimicrobial facemask and a process for manufacture of the facemask.

### BACKGROUND OF THE INVENTION

Recently, the global pandemic caused by the SARS-CoV-2 virus has highlighted the need for effective Personal Protective Equipment (PPE), both in social and professional environments. The protection of users afforded by the mask can prevent their infection with a virus, which could have negative health outcomes for the individual and wider negative impacts on society.

Facemasks are typically made up of multiple layers. These layers can be made from a variety of materials such as polypropylene, polyester or cellulose. A filtration element may trap infective agents such as bacterial and viruses, but unless they are rapidly killed they may grow and become a further contaminating source.

WO2004088029 discloses a paper mask with copper ions for viral inactivation.

WO2009146412 reports an antiviral mask comprising copper ions. The examples illustrate a requirement to chemically activate the fabric to bind the actives.

WO2010138426 details a mask for decreasing the transmission of pathogens. It is limited to polypropylene non-wovens. The claimed formulation as applied specifically includes polyvinyl alcohol, and polyethylene sorbitol as a non-ionic surfactant. The treated layer is specified to be an inner layer within a composite mask structure.

JP2010024587 claims the use of organic acid from 7 to 20 carbons for an antiviral masks. It claims the use of anionic surfactants.

JP2011042615 claims the antiviral properties of masks made from specific methacrylate polymers with copper ions.

CN111206417 discloses an antimicrobial cellulose fabric made by deposition of copper salts from alcoholic solutions.

WO2011040035 discloses particles of low solubility copper iodide bound to mask fabric via the requirement of a silane binder.

WO2012130117 claims a mask targeted to decrease pathogen transmission via a low pH hydrophilic layer and requires an organic acid.

WO2017073675 details an antiviral mask comprising copper ions made from knitted fabrics.

WO2018074337 disclosed an antimicrobial mask based on inorganic fine particles.

### SUMMARY OF THE INVENTION

The present invention identifies certain biocides that are suitable for depositing on fibres (for example polypropylene, polyethylene, polyester, nylon or cellulose) that may be used to construct PPE masks, and demonstrates their antimicrobial properties. In particular, the present invention provides an antimicrobial mask comprising certain biocides, in conjunction with certain surfactants.

The present invention provides an antimicrobial facemask comprising a non-woven fabric or material incorporating a water soluble copper salt and a zwitterionic surfactant or alkylsulfate surfactant. The alkylsulfate surfactant may also be an alkylsulfate salt surfactant. "Incorporating" can include the concepts of coated, impregnated and dyed.

The present invention further provides a process for treating a fabric suitable for a facemask construction with an aqueous solution of the desired formulation. The present invention therefore provides process for production of the facemask, wherein an active formulation of the water soluble copper salt and zwitterionic or alkylsulfate or salt surfactant is applied to the non-woven fabric as a solution in water.

The present invention also provides the use of the facemask as Personal Protective Equipment (PPE) i.e. exclusively to protect the user from infection.

### DETAILED DESCRIPTION OF THE INVENTION

The antimicrobial mask comprises at least one layer of nonwoven fabric, for example meltblown or spunbond formed polypropylene, polyethylene, polyester, cellulose or nylon, onto which is deposited the antimicrobial formulation. Preferably the mask is a composite layered mask i.e. multi-layered. Biocides are selected from aqueous soluble copper salts, or organic acids, or a combination of both. Surfactants are selected from zwitterionic surfactants or alkylsulfate surfactants or alkylsulfate salt surfactants.

The present invention takes advantage of the antimicrobial power of copper ions to protect users, but with increased efficacy from a novel formulation including zwitterionic surfactant or alkylsulfate or salt surfactant. When in a composite layered mask form, the water soluble copper salt and zwitterionic or alkylsulfate or salt surfactant are incorporated in the outer layer or an inner layer or both of the composite layered mask.

Preferably the copper salt has at a solubility of at least 100 g / litre of deionised water at 20 °C. Also preferred is wherein the copper salt is copper sulfate.

Preferably the zwitterionic surfactant comprises an alkyl chain of between 8 and 16 carbons. It is also preferred when the zwitterionic surfactant has a betaine type structure, preferably with quaternary alkyl at the nitrogen i.e. N-quaternary alkyl glycine. The most preferred zwitterionic surfactant is N,N-dimethyl-N-dodecylglycine betaine.

Alternatively preferable is an alkylsulfate surfactant or its salt comprising an alkyl chain of between 8 and 16 carbons, preferably sodium dodecylsulfate.

The facemask may also incorporate an organic carboxylic acid, preferably having between 3 and 7 carbons. Preferred acids include propionic acid, citric acid or lactic acid. Alternatively, the facemask may comprise no organic acid.

Preferably the non-woven fabric is cellulosic. In this respect the non-woven fabric facemask can comprise or consist essentially of cellulosic fibres, preferably at least 50 % by weight.

For manufacture of the facemask an active formulation of the water soluble copper salt and surfactant can be applied to the non-woven fabric as a solution in water. When in a composite layered mask form, the active formulation is applied to the outer layer or an inner layer or both of the composite layered mask.

The copper salt may be present in the active formulation in a concentration amount of 0.25 to 10 % wt/wt, preferably 0.5 to 5 % wt/wt. The surfactant may be present in a concentration amount of 0.05 to 5 % wt/wt, preferably 0.1 to 3 % wt. The organic acid where included may be present in a concentration amount of 0.1 to 5 % wt/wt, preferably 0.2 to 3 % wt/wt.

Surface active agents can significantly alter the surface energy of polymeric materials, aiding wetting and adhesion. However, this presents a key technical hurdle. When requiring a water soluble copper presentation to enable deposition onto fabric, many surfactants are unsatisfactory. Anionic surfactants (for example sodium dodecylbenzenesulfonate) have very low and insufficient solubility in solutions of copper sulfate. Amine oxide surfactants such as dimethyl dodecylamine N-oxide or N,N-dimethyloleyl N-oxide (CAS 14351-50-9) are also unacceptable, causing precipitation from copper sulfate solutions. Unexpectedly, it was found that zwitterionic betaine surfactants, such as dimethyl dodecylglycine betaine (CAS 66455-29-6) or lauryl sulfobetaine (CAS 14933-08-5) did not cause precipitation, and gave a homogeneous solution suitable for coating non-woven fabric. In addition we made the unexpected discovery that in sharp contrast to sodium dodecylbenzenesulfonate, the structurally related sodium dodecylsulfate also gave an acceptable solution for coating.

The present invention will now be illustrated, but in no way limited, by reference to the following examples.

### Example 1 - Fabric preparation

Copper sulfate (0.25 g) and citric acid (0.10 g) were dissolved in 24.65 g water, and a 18 x 18 cm square of non-woven cellulose fabric (suitable for facemask construction) added. The bath was heated in a 50 °C oven for 10 min, then the fabric removed and air dried.

### Example 2 - Fabric preparation

Copper sulfate (0.25 g), sodium dodecylsulfate (0.05 g) and citric acid (0.10 g) were dissolved in 24.60 g water, and a 18 x 18 cm square of non-woven cellulose fabric (suitable for facemask construction) added. The bath was heated in a 50 °C oven for 10 min, then the fabric removed and air dried.

### Example 3 - Fabric preparation

Copper sulfate (0.25 g), lauryl sulfobetaine (CAS 14933-08-5) (0.05 g) and citric acid (0.10 g) were dissolved in 24.60 g water, and a 18 x 18 cm square of non-woven cellulose fabric (suitable for facemask construction) added. The bath was heated in a 50 °C oven for 10 min, then the fabric removed and air dried.

### Example 4 - Fabric preparation

Copper sulfate (0.25 g), Empigen BB (0.10 g) and citric acid (0.10 g) were dissolved in 24.55 g water, and a 18 x 18 cm square of non-woven cellulose fabric (suitable for facemask construction) added. The bath was heated in a 50 °C oven for 10 min, then the fabric removed and air dried.

### Example 5 - Microbiology performance of above fabrics

A 4 cm disc of the sample prepared in Example 4 was inoculated with a 0.1 ml presentation of either Staphylococcus aureus ("Staph a") or Escherichia coli ("E coli"). After 1 h at 37 °C, a reduction of > 5 Log was achieved for both Staph a and E coli.

## Claims

1. An antimicrobial facemask comprising a non-woven fabric incorporating a water soluble copper salt and surfactant, wherein the surfactant is a zwitterionic surfactant or an alkylsulfate surfactant or an alkylsulfate salt surfactant.

2. The facemask according to claim 1, wherein the copper salt has at a solubility of at least 100 g / litre of deionised water at 20 °C.

3. The facemask according to claim 1 or 2, wherein the copper salt is copper sulfate

4. The facemask according to any preceding claim, wherein the zwitterionic surfactant comprises an alkyl chain of between 8 and 16 carbons.

5. The facemask according to any preceding claim, wherein the zwitterionic surfactant has a sulfobetaine type structure, preferably lauryl sulfobetaine.

6. The facemask according to any of claims 1 to 4, wherein the zwitterionic surfactant has a betaine type structure, preferably based on a trialkyl glycine.

7. The facemask according to any of claims 1 to 4, wherein the zwitterionic surfactant is N,N-dimethyl-N-dodecylglycine betaine.

8. The facemask according to any of claims 1 to 3, wherein the alkylsulfate or its salt comprises an alkyl chain of between 8 and 16 carbons, preferably wherein the alkylsulfate is sodium dodecylsulfate.

9. A facemask according to any preceding claim also incorporating an organic carboxylic acid.

10. A facemask according to claim 9, wherein the organic carboxylic acid has between 3 and 7 carbons.

11. A facemask according to claim 9, wherein the organic acid is any of propionic acid, citric acid or lactic acid.

12. A facemask according to any preceding claim, wherein the facemask is a composite layered mask, optionally wherein the water soluble copper salt and surfactant are incorporated in the outer layer and/or an inner layer of the composite layered mask.

13. A facemask according to any preceding claim, wherein the non-woven fabric is cellulosic, preferably wherein the non-woven fabric consists essentially of cellulosic fibres, preferably at least 50 % by weight.

14. Use of a facemask according to any preceding claim as Personal Protective Equipment.

15. A process for production of a facemask according to any preceding claim, wherein an active formulation of the water soluble copper salt and surfactant is applied to the non-woven fabric as a solution in water, preferably with the following concentration amounts:
a) copper salt at 0.25 - 10 % wt/wt, preferably 0.5 - 5 % wt/wt
b) surfactant at 0.05 - 5 % wt/wt, preferably 0.1 - 3 % wt/wt, and optionally when included
c) organic acid 0.1 - 5 % wt/wt, preferably 0.2 - 3 % wt/wt.
